(19) Europäisches Patentamt
European Patent Office
Office européen des brevets

(11) Veröffentlichungsnummer : **0 063 368**
**B1**

(12) # EUROPÄISCHE PATENTSCHRIFT

(45) Veröffentlichungstag der Patentschrift : **25.07.84**

(51) Int. Cl.³ : **C 07 J 7/00**, C 07 J 1/00, A 61 K 31/565

(21) Anmeldenummer : **82103220.8**

(22) Anmeldetag : **16.04.82**

(54) **Verfahren zur Herstellung von Pregn-4-en-3,20-dionderivaten sowie 17-alpha-Äthinyl-17-beta-trifluoracetoxygon-4-en-3-onderivate und die letzteren enthaltende Arzneimittel.**

(30) Priorität : **16.04.81 HU 98981**
**16.04.81 HU 98781**

(43) Veröffentlichungstag der Anmeldung :
**27.10.82 Patentblatt 82/43**

(45) Bekanntmachung des Hinweises auf die Patenterteilung : **25.07.84 Patentblatt 84/30**

(84) Benannte Vertragsstaaten :
**CH DE FR GB IT LI NL SE**

(56) Entgegenhaltungen :
**DE-B- 2 625 306**
**FR-A- 2 088 348**
**FR-A- 2 149 911**
**CHEMICAL ABSTRACTS, Band 53, Nr. 22, 25. November 1959, Spalte 22081f-i, Columbus, Ohio, USA JOJI YAMADA: "Long-acting progestational substances. 17alpha-nethyl-19-nortestosterone esters and 17alpha-ethynyltestosterone esters"**

(73) Patentinhaber : **RICHTER GEDEON VEGYESZETI GYAR R.T.**
**Gyömröi ut 19-21**
**H-1475 Budapest X (HU)**

(72) Erfinder : **Boòr, Anna, geb. Mezei**
**Mátyás király ut 4**
**H-1121 Budapest (HU)**
Erfinder : **Tòth, Jòzsef, Dr.**
**Alsò-Törökvész u. 10**
**Budapest II (HU)**
Erfinder : **Szén, Tamás, Dr.**
**Nagy Lajos király utja 151**
**H-1149 Budapest (HU)**
Erfinder : **Gábor, Lászlò**
**Gábor Aron u. 1/b**
**Budapest XVIII (HU)**
Erfinder : **Major, Piroska, geb. Forstner**
**Páskomliget u. 51/IX. 37**
**H-1156 Budapest (HU)**
Erfinder : **Holly, Sándor, Dr.**
**Városház u. 14**
**Budapest V (HU)**

(74) Vertreter : **Beszédes, Stephan G. Dr.**
**Münchener Strasse 80a Postfach 1168**
**D-8060 Dachau (DE)**

**EP 0 063 368 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Pregn-4-en-3,20-dionderivaten der allgemeinen Formel

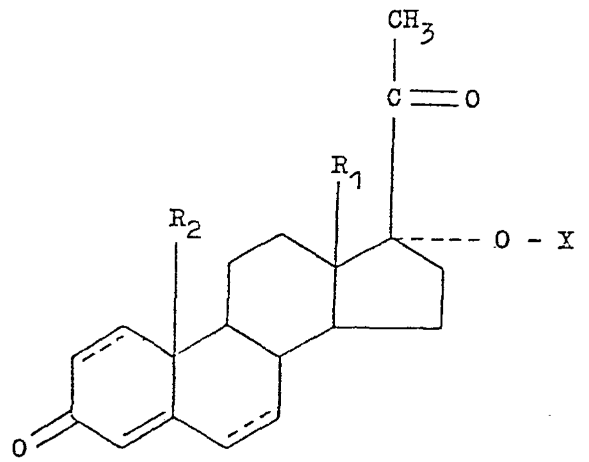

(I)

worin

$R_1$ für einen Methyl- oder Äthylrest steht,

$R_2$ Wasserstoff oder einen Methylrest bedeutet,

X einen Formyl- oder Acetylrest oder Wasserstoff darstellt und

die gestrichelten Linien (----) gegebenenfalls vorliegende weitere chemische Bindungen bedeuten, sowie pharmakologisch wirksame und neue Zwischenprodukte darstellende 17α-Äthinyl-17β-trifluoracetoxygon-4-en-3-onderivate und diese letzteren enthaltende Arzneimittel, insbesondere mit empfängnisverhütender Wirkung.

Verfahren zum Ausbilden der Pregnanseitenkette waren schon früher bekannt (Ber. 71, 1 487 ; Ber. 72 [1939], 182 ; Ber 97 [1964], 2 011 ; Helv. Chim. Acta 22 [1939], 755 ; J. Am. Chem. Soc. 81 [1959], 5 725 ; US-Patentschrift 4 041 055). Für eine technische beziehungsweise industrielle Durchführung sind diese Verfahren jedoch kaum oder überhaupt nicht geeignet. Gründe dafür sind unter anderem die zu große Zahl der Verfahrensstufen, die geringen Ausbeuten, die extremen Reaktionsbedingungen und in einigen Fällen die Unzugänglichkeit und schwierige Handhabbarkeit der zu verwendenden Reaktionsteilnehmer.

In den deutschen Offenlegungsschriften 2 140 291 und 2 230 286 und der entsprechenden französischen Offenlegungsschrift 2 149 911 sowie in der französischen Offenlegungsschrift 2 088 348 wurden als Katalysatoren bei der Ausbildung der Pregnanseitenkette Quecksilbersalze verwendet. Diese von 17α-Äthinyl-17β-sulfiten ausgehenden Verfahren liefern jedoch insbesondere bei größeren Ansätzen keine befriedigende Ausbeute und auch die Bildung des isomeren 17α-Pregnanderivates ist unvermeidlich. So beträgt die Ausbeute schon bei kleinen Ansätzen im Beispiel 7 der französischen Offenlegungsschrift 2 088 348 nur 26,8 % und im Beispiel 4 der französischen Offenlegungsschrift 2 149 911 nur 53 % bei starker Isomerenbildung.

Eine Weiterentwicklung der Umsetzung zum Ausbilden der Pregnanseitenkette unter Verwendung von Quecksilbersalzen als Katalysatoren ist in der ungarischen Patentschrift 174 982 und der entsprechenden deutschen Auslegeschrift 26 25 306 beschrieben. Dieses Verfahren geht von den 17β-Salpetersäureestern der 17α-Äthinyl-17β-hydroxygonane aus, als Katalysatoren werden Quecksilbersalze von niederen Carbonsäuren verwendet und die 17β-Salpetersäureester der 17α-Äthinyl-17β-hydroxygonane werden in dipolaren aprotonischen Medien oder basischen Lösungsmitteln mit Ameisensäure oder Essigsäure umgesetzt. Gemäß den Beispielen der Beschreibung dieser Druckschrift werden auf diese Weise Ausbeuten von 44,1 bis 83,0 % erhalten. Es ist jedoch zu bemerken, daß von den Verfassern dieser Druckschrift in einer späteren Veröffentlichung (Ber. 111 [1978], 3 086) für die Herstellung des 17α-Formyloxy-20-ketopregnanderivates nach diesem Verfahren Ausbeuten von nur 50 bis 70 % angegeben wurden, wobei auch noch auf in der technischen beziehungsweise industriellen Praxis kaum mögliche umständliche Weise chromatographisch isoliert werden mußte. Das 17α-Acetoxyprogesteron konnte von den Verfassern (statt der in der ungarischen Patentschrift 174 982 angegebenen Ausbeute von 40 %) sogar in einer Ausbeute von nur 6,4 % isoliert werden und diese Ausbeute ließ sich auch durch Zugabe von Hexamethylphosphorsäuretriamid und 48-stündiges Stehen nicht verbessern.

Das zuletzt beschriebene bekannte Verfahren hat zahlreiche Nachteile. Auf den Ablauf von Nebenreaktionen weist außer der mittelmäßigen Ausbeute auch der Umstand hin, daß in sämtlichen Beispielen der Beschreibung der ungarischen Patentschrift 174 982 die Reinigung des Rohproduktes durch Chromatographieren an Kieselgel vorgeschrieben ist, was arbeits- und lösungsmittelaufwendig ist und sich technisch beziehungsweise industriell nur schwer verwirklichen läßt. Der größte Nachteil des Verfahrens liegt jedoch darin, daß sich die als Ausgangsverbindungen dienenden 17β-Salpetersäureester

der 17α-Äthinyl-17β-hydroxygonane nur schwierig herstellen und schlecht handhaben lassen. So muß bei der Herstellung dieser Ausgangsverbindungen die Temperatur des Reaktionsgemisches auf − 20 °C gehalten werden. Die Ausbeuten sind niedrig ; von 17α-Äthinyl-17β-hydroxyandrost-4-en-3-on ausgehend wird der entsprechende 17β-Salpetersäureester in einer Ausbeute von 66 % erhalten (Ber. 111 [1978], 3 086). Die isolierten 17β-Salpetersäureester der 17α-Äthinyl-17β-hydroxygonane sind schwer zu handhabende, oft ölige, instabile Substanzen und ihre Herstellung und Verwendung wirft auch sicherheitstechnische Probleme auf. So ist zum Beispiel der 17α-Äthinyl-17β-hydroxyandrost-4-en-3-on-17-salpetersäureester, der unter anderem ein Zwischenprodukt der Herstellung des Prednisolones sein kann, eine explosive Substanz und nach eigenen Untersuchungen gefährlicher als das ausgesprochen als Sprengstoff verwendete 2,4,6-Trinitrotoluol (TNT). Im Gegensatz zur Aussage der ungarischen Patentschrift 174 982 ist demnach festzustellen, daß das Ausbilden der Pregnanseitenkette über die 17β-Salpetersäureester der 17α-Äthinyl-17β-hydroxy-steroide für die technische beziehungsweise industrielle Praxis kein gangbarer Weg ist.

Es wurde bereits die Trifluoracetylgruppe zum vorübergehenden Schutz von in der 11β-, 17α- und 21-Stellung befindlichen Hydroxygruppen bei bestimmten Steroiden mit Pregnangerüst verwendet (deutsche Auslegeschrift 2 144 405, US-Patentschrift 2 854 465 und britische Patentschrift 1 185 344), die Ester der 17α-Äthinyl-17β-hydroxygonane mit Trifluoressigsäure wurden jedoch bisher noch nicht beschrieben.

Ferner sind aus CHEMICAL ABSTRACTS, Band 53, Nr. 22, 25. November 1959, Spalte 22081f bis i 17α-Äthinyltestosteron und sein Essigsäureester und Capronsäureester sowie ihre progestationale Wirkung bekannt, wobei angegeben ist, daß die Follikelhormonwirksamkeit und ihre Dauer bei peroraler Verabreichung bei den Estern nicht höher als beim freien 17α-Äthinyltestosteron war.

Der Erfindung liegt die Aufgabe zugrunde, unter Behebung der Nachteile des Standes der Technik ein Verfahren zur Herstellung der Pregn-4-en-3,20-dionderivate der allgemeinen Formel I durch Ausbilden der Pregnanseitenkette unter Verwerten der bei der Steroidtotalsynthese als Zwischenprodukte anfallenden 17α-Äthinyl-17β-hydroxyderivate, welches auch technisch beziehungsweise industriell durchgeführt werden kann, indem von leicht herstellbaren und gut zu handhabenden Ausgangssubstanzen ausgegangen werden kann und keine Nebenreaktionen auftreten und somit reinere Produkte erhalten werden, sowie neue Zwischenprodukte zu dessen Durchführung, welche selbst wertvolle pharmakologische Wirkungen haben, und diese letzteren enthaltende Arzneimittel zu schaffen.

Das Obige wurde überraschenderweise durch die Erfindung erreicht.

Die Erfindung beruht auf der überraschenden Feststellung, daß zur Herstellung der 17α-Formyloxy- oder 17α-Acetoxypregn-4-en-3,20-dionderivate der allgemeinen Formel I die 17β-Trifluoressigsäureester der entsprechenden 17α-Äthinyl-17β-hydroxygon-4-en-3-onderivate oder anders ausgedrückt 17α-Äthinyl-17β-trifluoracetoxygon-4-en-3-on-derivate als Ausgangssubstanzen herangezogen werden können, weil diese bei ihrer Umsetzung mit Ameisensäure oder Essigsäure in Gegenwart von Quecksilbersalzen in dipolaren aprotonischen oder basischen Lösungsmitteln in praktisch quantitativen Ausbeuten die gewünschten 17α-Formyloxy- oder 17α-Acetoxypregn-4-en-3,20-dionderivate der allgemeinen Formel I liefern. Dabei haben die 17α-Äthinyl-17β-trifluor-acetoxygon-4-en-3-onderivate überraschenderweise selbst wertvolle pharmakologische, insbesondere empfängnisverhütende, Wirkungen.

Gegenstand der Erfindung ist daher ein Verfahren zur Herstellung von Pregn-4-en-3,20-dionderivaten der allgemeinen Formel

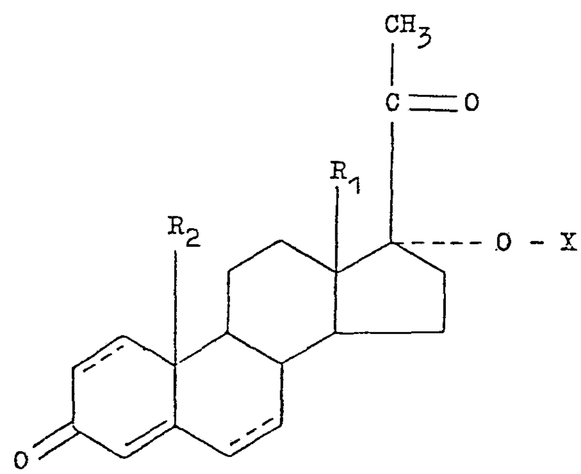

(I)

worin

$R_1$ für einen Methyl- oder Äthylrest steht,

$R_2$ Wasserstoff oder einen Methylrest bedeutet,

X einen Formyl- oder Acetylrest oder Wasserstoff darstellt und

die gestrichelten Linien (----) gegebenenfalls vorliegende weitere chemische Bindungen bedeuten,

durch Umsetzen von 17β-Estern von 17α-Äthinyl-17β-hydroxygonanderivaten mit Ameisensäure oder Essigsäure in Gegenwart von Quecksilbersalzen als Katalysatoren in dipolaren aprotonischen oder

basischen Lösungsmitteln, welches dadurch gekennzeichnet ist, daß als 17β-Ester von 17α-Äthinyl-17β-hydroxygonanderivaten 17α-Äthinyl-17β-trifluoracetoxygon-4-en-3-onderivate der allgemeinen Formel

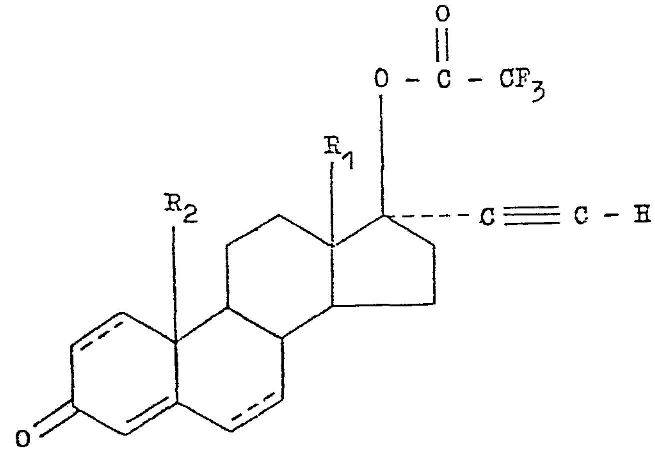

(II)

worin $R_1$ und $R_2$ sowie die gestrichelten Linien wie oben festgelegt sind, in Gegenwart von katalytischen Mengen von 1 oder mehr dissoziierenden Quecksilbersalz(en) in 1 oder mehr dipolaren aprotonischen (aprotischen) oder basischen Lösungsmittel(n) mit Ameisensäure oder Essigsäure umgesetzt werden und in an sich bekannter Weise gegebenenfalls von den erhaltenen Pregn-4-en-3,20-dionderivaten der allgemeinen Formel I, bei welchen X für einen Formyl- oder Acetylrest steht, dieser abgespalten wird.

Zweckmäßig wird beziehungsweise werden im erfindungsgemäßen Verfahren als dissoziirende[s] Quecksilbersalz(e) 1 oder mehr Quecksilber(I)-und/oder Quecksilber(II)-salz(e) von Carbonsäuren und/oder dissoziierende[s] anorganische[s] Quecksilber(I)- und/oder Quecksilber(II)-salz(e) verwendet.

Vorzugsweise wird beziehungsweise werden als Quecksilber(I)- und/oder Quecksilber(II)-salz(e) von Carbonsäuren Quecksilber(I)- und/oder Quecksilber(II)-acetat und/oder Quecksilber(II)-benzoat und/oder Quecksilber(II)-trifluoracetat verwendet.

Es ist auch bevorzugt, als dissoziierende[s] anorganische[s] Quecksilber(I)- und/oder Quecksilber(II)-salz(e) Quecksilber(II)-nitrat und/oder Quecksilber(I)-sulfat zu verwenden.

Vorzugsweise wird beziehungsweise werden im erfindungsgemäßen Verfahren als dipolare[s] aprotonische[s] Lösungsmittel Dimethylformamid, Dimethylacetamid, Dimethylsulfoxyd und/oder Hexamethylphosphorsäuretriamid verwendet.

Es ist bevorzugt, als basische[s] Lösungsmittel 1 oder mehr tertiäre[s] Amin(e), insbesondere Triäthylamin, zu verwenden. Weitere Beispiele für verwendbare tertiäre Amine sind Trimethylamin und N-Methylpiperidin.

Zweckmäßig wird die Umsetzung der 17α-Äthinyl-17β-trifluoracetoxygon-4-en-3-onderivate der allgemeinen Formel II mit der Ameisensäure oder Essigsäure bei 20 bis 80 °C durchgeführt. Das Arbeiten bei höheren oder tieferen Temperaturen ist nicht zu empfehlen, weil oberhalb 80 °C bereits mit Nebenreaktionen gerechnet werden muß und unterhalb 20 °C die Reaktionsgeschwindigkeit zu gering ist. Vorzugsweise werden Temperaturen von 45 bis 55 °C angewandt.

Das Reaktionsgemisch kann erforderlichenfalls mit inerten Lösungsmitteln, zum Beispiel Chloroform, Dioxan und/oder Acetonitril, verdünnt werden.

Vorteilhaft wird das gegebenenfalls erfolgende Abspalten des Formyl- beziehungsweise Acetylrestes von den erhaltenen Pregn-4-en-3,20-dionderivaten der allgemeinen Formel I, bei welchen X für einen Formyl- oder Acetylrest steht, durch Behandlung mit konzentrierter Salzsäure und Methanol durchgeführt.

Die im erfindungsgemäßen Verfahren als Ausgangssubstanzen verwendeten 17α-Äthinyl-17β-trifluoracetoxygon-4-en-3-onderivate der allgemeinen Formel II können in der Weise hergestellt werden, daß 17α-Äthinyl-17β-hydroxygon-4-en-3-onderivate der allgemeinen Formel

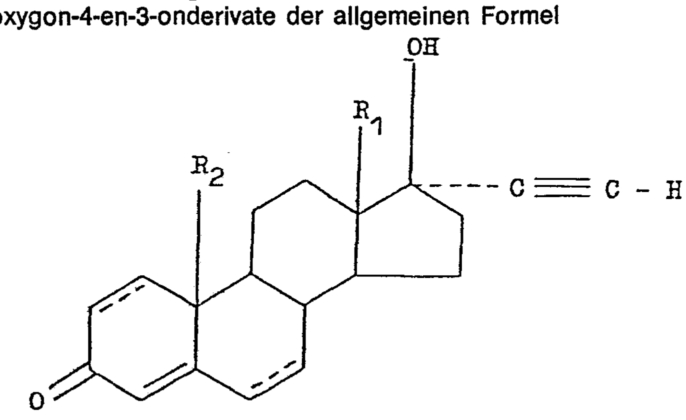

(III)

worin $R_1$ und $R_2$ sowie die gestrichelten Linien wie oben festgelegt sind, in Gegenwart von säurebindenden Mitteln mit Trifluoressigsäureanhydrid umgesetzt werden (Trifluoracetylierung). [Dieses Verfahren gehört nicht dem Stand der Technik an und ist Gegenstand der der nur der ungarischen Patentanmeldung mit dem Aktenzeichen 987/81 vom 16. April 1981 entsprechenden gleichzeitig eingereichten europäischen Patentanmeldung der Anmelderin EP-A-82 103 221.6/63 369].

Die im erfindungsgemäßen Verfahren als Ausgangsverbindungen dienenden 17α-Äthinyl-17β-trifluoracetoxygon-4-en-3-onderivate der allgemeinen Formel II können auch in situ hergestellt werden. In diesem Falle wird das Produkt der Trifluoracetylierung nicht isoliert, sondern im gleichen Reaktionsgemisch in Gegenwart von Quecksilbersalzen mit Ameisensäure oder Essigsäure umgesetzt und gegebenenfalls vom erhaltenen Pregn-4-en-3,20-dionderivatprodukt der allgemeinen Formel I, bei welchem X für einen Formyl- oder Acetylrest steht, also vom entsprechenden 17α-Formyloxy-beziehungsweise 17α-Äcetyloxypregn-4-en-3,20-dionderivatprodukt, dieser Umsetzung ebenfalls ohne zwischenzeitliche Isolierung der Formyl- beziehungsweise Acetylrest, vorteilhaft durch Behandlung mit konzentrierter Salzsäure und Methylalkohol, abgespalten, wodurch unmittelbar das entsprechende Pregn-4-en-3,20-dionderivat der allgemeinen Formel I, bei welchem X für Wasserstoff steht, also das entsprechende 17α-Hydroxypregn-4-en-3,20-dionderivat, erhalten wird.

Nach einer vorteilhaften Ausführungsform des erfindungsgemäßen Verfahrens werden daher als 17α-Äthinyl-17β-trifluoracetoxygon-4-en-3-onderivate der allgemeinen Formel II solche in den bei ihrer Herstellung aus den entsprechenden 17α-Äthinyl-17β-hydroxygon-4-en-3-onderivaten angefallenen Reaktionsgemischen in situ eingesetzt.

Das erfindungsgemäße Verfahren ist als ausgesprochen überraschend anzusehen, da nach Schrifttumsangaben und eigenen Untersuchungen die sonstigen 17β-Ester der 17α-Äthinyl-17β-hydroxygonane ein völlig anderes Verhalten zeigen. In der ungarischen Patentschrift 174 982 ist angegeben, daß bei der ähnlichen Umsetzung der 17β-Schwefligsäureester auch das 17α-Pregnanisomer in bedeutender Menge gebildet wird. Gemäß derselben Patentschrift ist bei Verwendung der 17β-Salpetersäureester die Ausbeute mittelmäßig (44 bis 83 % [in Wirklichkeit ist sie wie bereits erwähnt noch viel geringer]), und zwar, wie es anzunehmen ist, infolge von Nebenreaktionen. Bei eigenen Untersuchungen in dieser Richtung wurde festgestellt, daß die Ester der 17α-Äthinyl-17β-hydroxygonane mit Ameisensäure, Essigsäure und Monochloressigsäure in analoger Reaktion nahezu quantitativ zu 17α-Pregnanderivaten umgesetzt werden. Zur Herstellung der Pregnanderivate mit der gewünschten räumlichen Stellung der Seitenkette sind also nur die 17β-Trifluoressigsäureester der 17α-Äthinyl-17β-hydroxygonane geeignet.

Durch das erfindungsgemäße Verfahren können die 17α-Formyloxy- beziehungsweise 17α-Äcetoxypregn-4-en-3,20-dion-derivate der allgemeinen Formel I in sehr guten Ausbeuten, welche denen der durch die bekannten Verfahren erreichten überlegen sind, erhalten werden. Die Reaktionsbedingungen und die Reaktionsteilnehmer gewährleisten eine wirtschaftliche Durchführung des erfindungsgemäßen Verfahrens im technischen beziehungsweise industriellen Maßstab, welche nicht durch Sicherheitsbestimmungen beeinträchtigt wird. Vom Gesichtspunkt der Durchführung im Betriebsmaßstab ist es ein großer Vorteil, daß die als Ausgangsstoffe eingesetzten 17β-Trifluoressigsäureester der 17α-Äthinyl-17β-trifluoracetoxygon-4-en-3-onderivate der allgemeinen Formel II ohne zwischenzeitliche Isolierung erfindungsgemäß zu den entsprechenden 17α-Formyloxy- beziehungsweise 17α-Acetyl-oxypregn-4-en-3,20-dionderivaten der allgemeinen Formel I, ja sogar im selben Reaktionsgemisch noch, vorzugsweise durch Behandeln mit Salzsäure und Methylalkohol, zu den entsprechenden 17α-Hydroxypregn-4-en-3,20-dionderivaten der allgemeinen Formel I umgesetzt werden können.

Das erfindungsgemäße Verfahren bietet also einen neuen vorteilhaften Weg zur Ausbildung der für die Corticoide charakteristischen Pregnanseitenkette.

Das erfindungsgemäße Verfahren kann technisch beziehungsweise industriell sicher, leicht und wirtschaftlich durchgeführt werden, wobei zur Herstellung der als Ausgangssubstanzen zur Herstellung der Ausgangssubstanzen des erfindungsgemäßen Verfahrens dienenden 17α-Äthinyl-17β-hydroxy-4-en-3-onderivate der allgemeinen Formel III entweder von durch Abbau der Seitenkette von Sterinen (zum Beispiel Cholesterin, Sitosterin oder Stigmasterin) erhaltenen oder durch Totalsynthese erhaltenen 17-Oxo-androstanen ausgegangen werden kann, welche äthinyliert werden.

Die erfindungsgemäß hergestellten Pregn-4-en-3,20-dionderivate der allgemeinen Formel I haben eine wertvolle pharmakologische, insbesondere progestative, Wirksamkeit (vergleiche The Merck Index, 9. Auflage, Nr. 4 756). Sie sind auch wertvolle Zwischenprodukte zur in an sich bekannter Weise erfolgenden Herstellung von zahlreichen in der Pharmazie sehr wichtigen Wirkstoffen, zum Beispiel des gestagen wirkenden 17α-Hydroxyprogesteroncapronates (Hormofort®). Ferner sind die durch das erfindungsgemäße Verfahren hergestellten Pregn-4-en-3,20-dion-derivate der allgemeinen Formel I wichtige Zwischenprodukte der Reichstein-S-Synthese, das heißt zur in an sich bekannter Weise erfolgenden Herstellung von entzündungshemmenden Corticoiden, zum Beispiel Hydrocortison, Prednisolon, 11β,17α-Dihydroxy-21-(4′-methylpiperazin-1′-yl)-pregna-1,4-dien-3,20-dion (Depersolon®) und 9-Fluor-16α-hydroxyprednisolon (Triamcinolon) beziehungsweise seinem Acetonid.

Die im erfindungsgemäßen Verfahren als Ausgangssubstanzen verwendeten 17α-Äthinyl-17β-trifluoracetoxygon-4-en-3-onderivate der allgemeinen Formel II sind im Schrifttum bisher noch nicht beschriebene neue Verbindungen. Durch diese neuen Zwischenprodukte, welche wie bereits erwähnt selbst pharmakologisch, insbesondere zur Empfängnisverhütung, wirksam sind, wird ein neuer Weg durch ein

chemisch eigenartiges Verfahren zur Herstellung der pharmazeutisch wertvollen Substanzen Pregn-4-en-3,20-dionderivate der allgemeinen Formel I und über diese zur Herstellung der ebenfalls pharmazeutisch wertvollen Substanzen 17α-Hydroxyprogesteroncapronat und Corticoide, wie Hydrocortison, Prednisolon, 11β,17α-Dihydroxy-21-(4'-methylpiperazin-1'-yl)-pregna-1,4-dien-3,20-dion und 9-Fluor-16α-hydroxyprednisolon, der technisch fortschrittlich ist, eröffnet. Dies ist deswegen besonders überraschend, weil die aus CHEMICAL ABSTRACTS, Band 53, Nr. 22, 25. November 1959, Spalte 22081f bis i bekannten Verbindungen sich nicht zur Herstellung der Pregn-4-en-3,20-dionderivate der allgemeinen Formel I eignen, so daß die letzteren aus den ersteren beziehungsweise über die ersteren nicht hergestellt werden können.

Gegenstand der Erfindung sind daher auch 17α-Äthinyl-17β-trifluoracetoxygon-4-en-3-onderivate der allgemeinen Formel

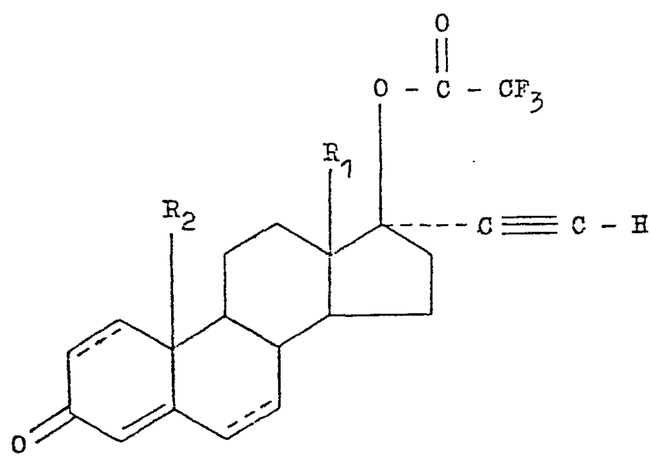

(II)

worin

$R_1$ für einen Methyl- oder Äthylrest steht,

$R_2$ Wasserstoff oder einen Methylrest bedeutet und

die gestrichelten Linien (----) gegebenenfalls vorliegende weitere chemische Bindungen bedeuten.

Es folgen Seiten 15 bis 21 der ursprünglichen Unterlagen vom 16. April 1982.

Bevorzugte erfindungsgemäße 17α-Athinyl-17β-trifluoracetoxygon-4-en-3-onderivate der allgemeinen Formel II sind 17α-Äthinyl-17β-trifluoracetoxyandrost-4-en-3-on, 17α-Äthinyl-17β-trifluoracetoxyandrosta-1,4-dien-3-on, 17α-Äthinyl-17β-trifluoracetoxyandrosta-1,4,6-trien-3-on, 17α-Äthinyl-17β-trifluoracetoxy-18-methylöstr-4-en-3-on und 17α-Äthinyl-17β-trifluoracetoxyöstr-4-en-3-on.

Ferner sind erfindungsgemäß Arzneimittel, welche 1 oder mehr der erfindungsgemäßen 17α-Äthinyl-17β-trifluoracetoxygon-4-en-3-onderivate der allgemeinen Formel II als Wirkstoff(e), zweckmäßig zusammen mit 1 oder mehr pharmazeutischen Konfektionierungsmittel(n), enthalten, vorgesehen.

Die Erfindung wird an Hand der folgenden Beispiele näher erläutert.

## Beispiel 1

400 ml konzentrierte Ameisensäure werden unter Eiskühlung mit 200 ml Hexamethylphosphorsäuretriamid vermischt. Das Gemisch wird mit 40,8 g (0,1 Mol) 17α-Äthinyl-17β-trifluoracetoxy-androst-4-en-3-on und 6,12 g Quecksilber(II) acetat versetzt, auf 50-55 °C erwärmt und bei dieser. Temperatur 3 Stunden lang gerührt. Dann wird das Reaktionsgemisch in 4 Liter Wasser gegossen, das ausgeschiedene Produkt wird abfiltriert, mit Wasser gewaschen und dann getrocknet. 34,0 g (95,0 %) 17α-Formyloxy-pregn-4-en-3,20-dion werden erhalten. Das aus Methylalkohol kristallisierte Produkt schmilzt bei 217-219 °C $[\alpha]_D^{20} = + 87,0°$ (c = 1 %, Chloroform).

## Beispiel 2

400 ml konzentrierte Ameisensäure und 300 ml Triäthylamin werden unter Eiskühlung miteinander vermischt. Das Gemisch wird mit 40,8 g (0,1 Mol) 17α-Äthinyl-17β-trifluoracetoxy-androst-4-en-3-on und 6,12 g Quecksilber(II) acetat versetzt, bei 55 °C 4 Stunden lang gerührt und dann in 4 Liter Wasser gegossen. Das ausgeschiedene Produkt wird abfiltriert und getrocknet. 34,3 g (95,8 %) 17α-Formyloxy-

pregn-4-en-3,20-dion werden erhalten. Das aus Methanol umkristallisierte Produkt schmilzt bei 218-221 °C.

Beispiel 3

40,8 g (0,1 Mol) 17α-Äthinyl-17β-trifluoracetoxy-androst-4-en-3-on werden auf die im Beispiel 1 beschriebene Weise umgesetzt, statt des Hexamethylphosphorsäuretriamides werden jedoch 300 ml Dimethylformamid verwendet. 33,6 g (93,8 %) rohes 17α-Formyloxy-pregn-4-en-3,20-dion werden erhalten, das nach Umkristallisieren aus Methanol bei 217-220 °C schmilzt. $[\alpha]_D^{20} = + 84{,}6°$ (c = 1 %, Chloroform).

Beispiel 4

4,08 g (0,01 Mol) 17α-Äthinyl-17β-trifluoracetoxy-androst-4-en-3-on werden in einem Gemisch aus 40 ml Ameinsensäure und 20 ml Hexamethylphosphorsäuretriamid auf die im Beispiel 1 beschriebene Weise umgesetzt, als Katalysator werden jedoch 0,62 g Quecksilber(II)nitrat zugesetzt. Nach der Aufarbeitung erhält man 3,35 g (93,6 %) 17α-Formyloxy-pregn-4-en-3,20-dion, das nach Umkristallisieren aus Methanol bei 218-221 °C schmilzt. $[\alpha]_D^{20} = + 88{,}3°$ (c = 1 %, Chloroform).

Beispiel 5

12 ml Essigsäure werden unter Eiskühlung mit 5 ml Dimethylformamid vermischt, mit 1,00 g (2,4 mMol) 17α-Äthinyl-17β-trifluoracetoxy-androst-4-en-3-on und 0,24 g Quecksilber(II)-acetat versetzt, auf 80 °C erwärmt und bei dieser Temperatur 10 Stunden lang gerührt. Dann wird das Gemisch in Wasser gegossen, die ausgeschiedenen Kristalle werden abfiltriert, mit Wasser gewaschen und dann getrocknet. 0,75 g (84,6 %) 17α-Acetoxy-pregn-4-en-3,20-dion werden erhalten. Das Produkt wird aus einem Aceton-Hexan-Gemisch (durch Lösen in Aceton und Fällen durch Zugabe von Hexan) umkristallisiert und schmilzt dann bei 240-244 °C. $[\alpha]_D^{20} = + 69{,}3°$ (c = 1 %, Chloroform).

Beispiel 6

10 ml Ameisensäure und 10 ml Dimethylformamid werden unter Eiskühlung miteinander vermischt. Das Gemisch wird mit 1,00 g (2,4 mMol) 17α-Äthinyl-17β-trifluoracetoxy-androsta-1,4-dien-3-on und 0,15 g Quecksilber(II) acetat versetzt, bei 60 °C 10 Stunden lang gerührt und dann in Wasser gegossen. Die ausgeschiedene Substanz wird abfiltriert, mit Wasser gewaschen und dann getrocknet. 0,78 g (91,8 %) 17α-Formyloxy-pregna-1,4-dien-3,20-dion werden erhalten, das nach Umkristallisieren aus Methanol bei 198-201 °C schmilzt.

Beispiel 7

12 ml Ameisensäure und 10 ml Dimethylformamid werden unter Eiskühlung miteinander vermischt. Das Gemisch wird mit 1,00 g (2,4 mMol) 17α-Äthinyl-17β-trifluoracetoxy-18-methyl-östr-4-en-3-on und 0,24 g Quecksilber(II) acetat versetzt, bei 45-50 °C 3 Stunden lang gerührt und dann in Wasser gegossen. Die ausgeschiedene kristalline Substanz wird abfiltriert, mit Wasser neutral gewaschen und dann getrocknet. 0,74 g (84,3 %) 17α-Formyloxy-18-methyl-19-nor-pregn-4-en-3,20-dion werden erhalten, das nach Umkristallisieren aus einem Aceton-Äther-Gemisch (durch Lösen in Aceton und Fällen durch Zugabe von Äther) bei 193-195 °C schmilzt. $[\alpha]_D^{20} = + 31{,}0°$ (c = 1 %, Chloroform).

Beispiel 8

12 ml Ameisensäure und 10 ml Dimethylformamid werden unter Kühlung miteinander vermischt. Das Gemisch wird mit 1 g (2,5 mMol) 17α-Äthinyl-17β-trifluoracetoxy-östr-4-en-3-on und 0,27 g Quecksilber(II)-acetat versetzt, bei 50 °C 3 Stunden lang gerührt und dann in Eiswasser gegossen. Die ausgeschiedene kristalline Substanz wird abfiltriert, gewaschen und getrocknet. 0,80 g (94,4 %) 17α-Formyloxy-19-nor-pregn-4-en-3,20-dion werden erhalten, das nach Umkristallisieren aus Methanol bei 201-203 °C schmilzt. $[\alpha]_D^{20} = + 32{,}0°$ (c = 1 %, $CHCl_3$).

Beispiel 9

Zu der Lösung von 3,12 g (0,01 Mol) 17α-Äthinyl-17β-hydroxyandrost-4-en-3-on in 31 ml Dimethylformamid werden bei 0-5 °C 2,09 ml (0,015 Mol) Trifluoressigsäureanhydrid gegeben. Das Gemisch wird bei der angegebenen Temperatur 10 Minuten lang gerührt. Dann wird das Gemisch mit 31 ml Ameisensäure und 0,46 g Quecksilber(II) acetat versetzt, bei 50-55 °C 9 Stunden lang gerührt und dann in Wasser gegossen. Die ausgefallene Substanz wird abfiltriert, gewaschen und getrocknet. 3,40 g (94,9 %) 17α-Formyloxy-pregn-4-en-3,20-dion werden erhalten. Das aus Methanol umkristallisierte Produkt schmilzt bei 217-219 °C.

Beispiel 10

3,12 g (0,01 Mol) 17α-Äthinyl-17β-hydroxy-androst-4-en-3-on werden auf die im Beispiel 9 beschriebene Weise umgesetzt. Nach der durch Quecksilber(II)acetat katalysierten Reaktion wird das Reaktionsgemisch jedoch nicht in Wasser gegossen, sondern mit dem Gemisch von 24 ml konzentrierter Salzsäure und 40 ml Methanol versetzt. Anschließend wird das Gemisch bei 48-50 °C 8 Stunden lang gerührt und dann in Wasser gegossen. Das ausgeschiedene Produkt wird abfiltriert, mit Wasser gewaschen und dann getrocknet. 2,70 g (82,1 %) 17α-Hydroxy-pregn-4-en-3,20-dion werden erhalten, das nach Umkristallisieren aus Methanol bei 212-217 °C schmilzt. $[\alpha]_D^{20} = +89°$ (c = 1 %, Chloroform).

**Ansprüche**

1. Verfahren zur Herstellung von Pregn-4-en-3,20-dion-derivaten der allgemeinen Formel

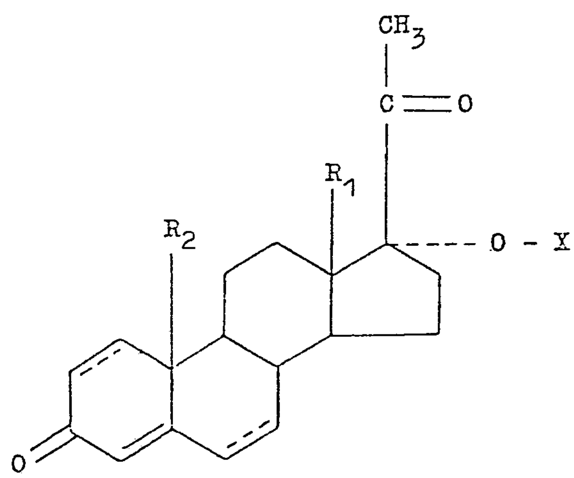

(I)

worin

$R_1$ für einen Methyl- oder Äthylrest steht,

$R_2$ Wasserstoff oder einen Methylrest bedeutet,

X einen Formyl- oder Acetylrest oder Wasserstoff darstellt und

die gestrichelten Linien (----) gegebenenfalls vorliegende weitere chemische Bindungen bedeuten,

durch Umsetzen von 17β-Estern von 17α-Äthinyl-17β-hydroxygonanderivaten mit Ameisensäure oder Essigsäure in Gegenwart von Quecksilbersalzen als Katalysatoren in dipolaren aprotonischen oder basischen Lösungsmitteln, dadurch gekennzeichnet, daß man als 17β-Ester von 17α-Äthinyl-17β-hydroxygonanderivaten 17α-Äthinyl-17β-trifluoracetoxygon-4-en-3-on-derivate der allgemeinen Formel

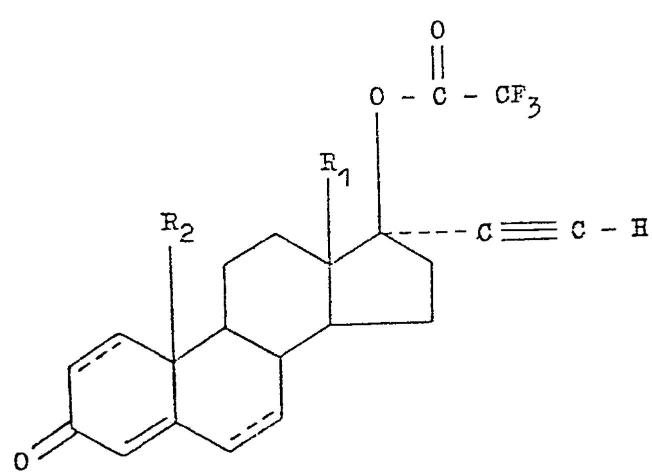

(II)

worin $R_1$ und $R_2$ sowie die gestrichelten Linien wie oben festgelegt sind, in Gegenwart von katalytischen Mengen von 1 oder mehr dissoziierenden Quecksilbersalz(en) in 1 oder mehr dipolaren aprotonischen

oder basischen Lösungsmittel(n) mit Ameisensäure oder Essigsäure umsetzt und in an sich bekannter Weise gegebenenfalls von den erhaltenen Pregn-4-en-3,20-dionderivaten der allgemeinen Formel I, bei welchen X für einen Formyl- oder Acetylrest steht, diesen abspaltet.

2. Verfahren nach Anspruch 1, dadurch gekennzeichnet, daß man als dissoziierende[s] Quecksilbersalz(e) 1 oder mehr Quecksilber(I)- und/oder Quecksilber(II)-salz(e) von Carbonsäuren und/oder dissoziierende[s] anorganische[s] Quecksilber(I)- und/oder Quecksilber(II)-salze(e) verwendet.

3. Verfahren nach Anspruch 1 oder 2, dadurch gekennzeichnet, daß man als Quecksilber(I)- und/oder Quecksilber(II)-salz(e) von Carbonsäuren Quecksilber(I)- und/oder Quecksilber(II)-acetat und/oder Quecksilber(II)-benzoat und/oder Quecksilber(II)-trifluoracetat verwendet.

4. Verfahren nach Anspruch 1 bis 3, dadurch gekennzeichnet, daß man als dissoziierende[s] anorganische[s] Quecksilber(I)- und/oder Quecksilber(II)-salz(e) Quecksilber(II)-nitrat und/oder Quecksilber(I)-sulfat verwendet.

5. Verfahren nach Anspruch 1 bis 4, dadurch gekennzeichnet, daß man als dipolare[s] aprotonische[s] Lösungsmittel Dimethylformamid, Dimethylacetamid, Dimethylsulfoxyd und/oder Hexamethylphosphorsäuretriamid verwendet.

6. Verfahren nach Anspruch 1 bis 4, dadurch gekennzeichnet, daß man als basische[s] Lösungsmittel 1 oder mehr tertiäre[s] Amin(e), insbesondere Triäthylamin, verwendet.

7. Verfahren nach Anspruch 1 bis 6, dadurch gekennzeichnet, daß man die Umsetzung der 17α-Äthinyl-17β-trifluoracetoxygon-4-en-3-onderivate der allgemeinen Formel II mit der Ameisensäure oder Essigsäure bei 20 bis 80 °C durchführt.

8. Verfahren nach Anspruch 1 bis 7, dadurch gekennzeichnet, daß man die Umsetzung der 17α-Äthinyl-17β-trifluoracetoxygon-4-en-3-onderivate der allgemeinen Formel II mit der Ameisensäure oder Essigsäure bei 45 bis 55 °C durchführt.

9. Verfahren nach Anspruch 1 bis 8, dadurch gekennzeichnet, daß man als 17α-Äthinyl-17β-trifluoracetoxygon-4-en-3-onderivate der allgemeinen Formel II solche in den bei ihrer Herstellung aus den entsprechenden 17α-Äthinyl-17β-hydroxygon-4-en-3-onderivaten angefallenen Reaktionsgemischen in situ einsetzt.

10. 17α-Äthinyl-17β-trifluoracetoxygon-4-en-3-onderivate der allgemeinen Formel

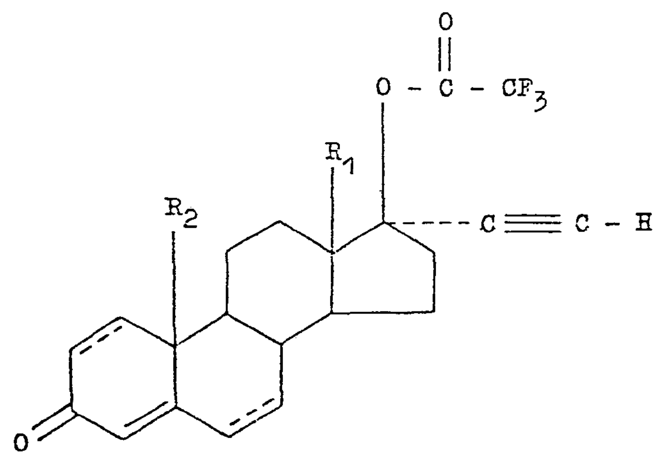

(II)

worin

$R_1$ für einen Methyl- oder Äthylrest steht,

$R_2$ Wasserstoff oder einen Methylrest bedeutet,

die gestrichelten Linien (----) gegebenenfalls vorliegende weitere chemische Bindungen bedeuten.

11. 17α-Äthinyl-17β-trifluoracetoxyandrost-4-en-3-on.

12. 17α-Äthinyl-17β-trifluoracetoxyandrosta-1,4-dien-3-on.

13. 17α-Äthinyl-17β-trifluoracetoxyandrosta-1,4,6-trien-3-on.

14. 17α-Äthinyl-17β-trifluoracetoxy-18-methylöstr-4-en-3-on.

15. 17α-Äthinyl-17β-trifluoracetoxyöstr-4-en-3-on.

16. Arzneimittel, gekennzeichnet durch einen Gehalt an 1 oder mehr Verbindung(en) nach Anspruch 10 bis 15 als Wirkstoff(en), zweckmäßig zusammen mit 1 oder mehr üblichen pharmazeutischen Konfektionierungsmittel(n).

**Claims**

1. A process for preparing pregn-4-ene-3,20-dione derivatives having the general formula

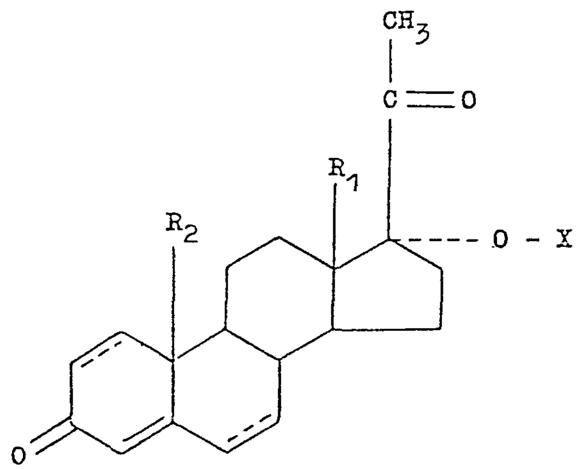

(I)

wherein

$R_1$ represents a methyl or ethyl radical,

$R_2$ means hydrogen or a methyl radical,

X represents a formyl or acetyl radical or hydrogen and

the dotted lines (----) mean further chemical bonds optionally present

by reacting 17β-esters of 17α-ethinyl-17β-hydroxy-gonane derivatives with formic acid or acetic acid in the presence of mercury salts as catalysts in dipolar aprotic or basic solvents, characterized in that one reacts as 17β-esters of 17α-ethinyl-17β-hydroxygonane derivatives 17α-ethinyl-17β-trifluoroacetoxygon-4-ene-3-one derivatives having the general

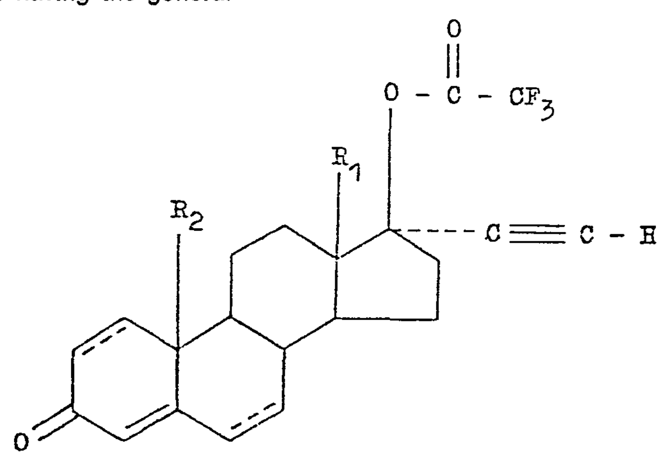

(II)

wherein $R_1$ and $R_2$ as well as the dotted lines are as above defined, in the presence of catalytic amounts of 1 or more dissociating mercury salt(s) in 1 or more dipolar aprotic or basic solvent(s) with formic acid or acetic acid and in a manner known per se optionally from the obtained pregn-4-ene-3,20-dione derivatives having the general formula I in which X represents a formyl or acetyl radical one splits off this.

2. A process according to claim 1, characterized in that one uses as [a] dissociating mercury salt(s) 1 or more mercury(I) and/or mercury(II)-salt(s) of carboxylic acids and/or dissociating inorganic mercury(I)- and/or mercury(II)-salt(s).

3. A process according to claim 1 or 2, characterized in that one uses as mercury(I)- and/or mercury(II)-salt(s) of carboxylic acids mercury(I)- and/or mercury(II)-acetate and/or mercury(II)-benzoate and/or mercury(II)-trifluoroacetate.

4. A process according to claims 1 to 3, characterized in that one uses as [a] dissociating inorganic mercury(I)- and/or mercury(II)-salt(s) mercury(II)-nitrate and/or mercury(I)-sulphate.

5. A process according to claims 1 to 4, characterized in that one uses as [a] dipolar aprotic solvent(s) dimethylformamide, dimethylacetamide, dimethylsulphoxyde and/or hexamethylphosphoric acid triamide.

6. A process according to claims 1 to 4, characterized in that one uses as [a] basic solvent(s) 1 or more tertiary amine(s), particularly triethylamine.

7. A process according to claims 1 to 6, characterized in that one carries out the reaction of the 17α-ethinyl-17β-trifluoroacetoxygon-4-ene-3-one derivatives having the general formula II with the formic acid or acetic acid at 20 to 80 °C.

8. A process according to claims 1 to 7, characterized in that one carries out the reaction of the 17α-ethinyl-17β-trifluoroacetoxygon-4-ene-3-one derivatives having the general formula II with the formic acid

or acetic acid at 45 to 55 °C.

9. A process according to claims 1 to 8, characterized in that one employs as 17α-ethinyl-17β-trifluoroacetoxygon-4-ene-3-one derivatives having the general formula II such in situ in the reaction mixtures obtained in their preparation from the corresponding 17α-ethinyl-17β-hydroxygon-4-ene-3-one derivatives.

10. 17α-ethinyl-17β-trifluoroacetoxygon-4-ene-3-one derivatives having the general formula

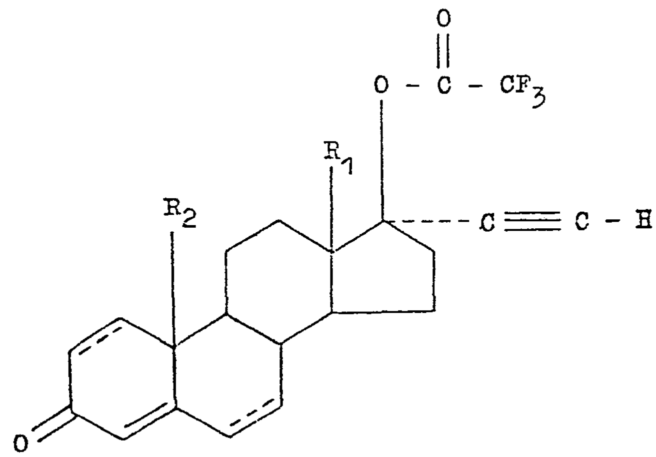

(II)

wherein

$R_1$ represents a methyl or ethyl radical,

$R_2$ means hydrogen or methyl radical and

the dotted lines (----) mean further chemical bonds optionally present.

11. 17α-ethinyl-17β-trifluoroacetoxyandrost-4-ene-3-one.

12. 17α-ethinyl-17β-trifluoroacetoxyandrosta-1,4-diene-3-one.

13. 17α-ethinyl-17β-trifluoroacetoxyandrosta-1,4,6-triene-3-one.

14. 17α-ethinyl-17β-trifluoroacetoxy-18-methyloestr-4-ene-3-one.

15. 17α-ethinyl-17β-trifluoroacetoxyoestr-4-ene-3-one.

16. Medicaments, characterized by a content of 1 or more compound(s) according to claims 10 to 15 as active principle(s), suitably together with 1 or more usual pharmaceutical processing agent(s).

**Revendications**

1. Procédé de préparation de dérivés de pregn-4-ène-3,20-dione de formule générale I

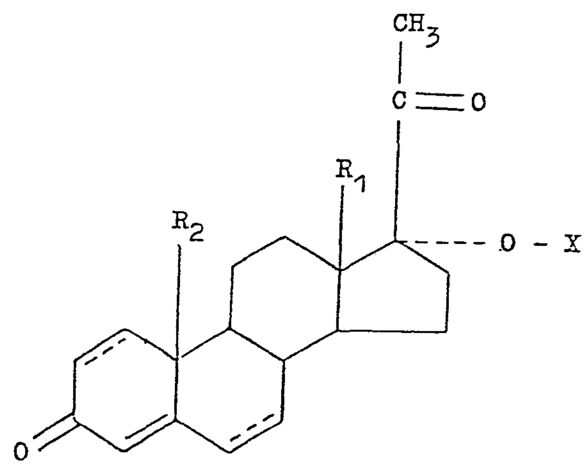

(I)

dans laquelle

$R_1$ représente un radical méthyle ou éthyle,

$R_2$ représente l'hydrogène ou un radical méthyle,

X représente un radical formyle ou acétyle ou l'hydrogène, et

les tiretés (----) représentent des liaisons chimiques supplémentaires existant éventuellement, par réaction de 17β-esters de dérivés 17α-éthynyl-17β-hydroxygonane sur l'acide formique ou l'acide acétique en présence de sels de mercure comme catalyseurs dans des solvants aprotiques ou basiques dipolaires, caractérisé par le fait que, comme 17β-esters de dérivés 17α-éthynyl-17β-hydroxygonane, on

fait réagir sur l'acide formique ou l'acide acétique des dérivés 17α-éthynyl-17β-trifluoracétoxygon-4-ène-3-one de formule générale II

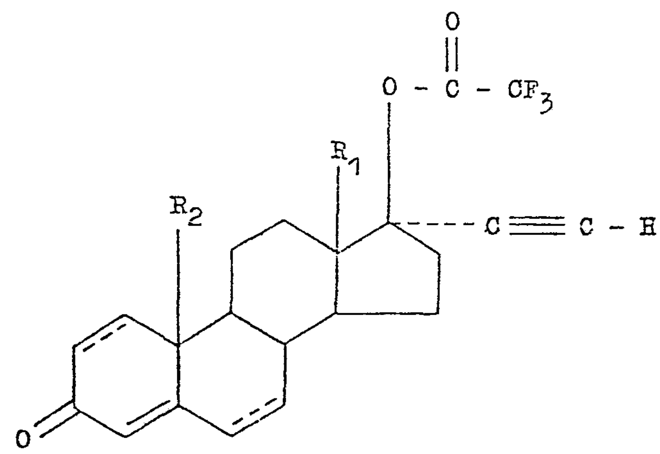

(II)

dans laquelle $R_1$ et $R_2$ ainsi que les tiretés sont tels que définis ci-dessus, en présence de quantités catalytiques d'un ou plusieurs sels de mercure dissociables dans un ou plusieurs solvants aprotiques ou basiques dipolaires, et qu'éventuellement on sépare celui-ci de manière en elle-même connue, des dérivés de pregn-4-ène-3,20-dione de formule générale I dans lesquels X représente un radical formyle ou acétyle.

2. Procédé selon la revendication 1, caractérisé par le fait que comme sels de mercure dissociables, on utilise un ou plusieurs sels de mercure (I) et/ou de mercure (II) d'acides carboxyliques et/ou sels minéraux dissociables de mercure (I) et/ou de mercure (II).

3. Procédé selon l'une des revendications 1 et 2, caractérisé par le fait que comme sels de mercure (I) et/ou de mercure (II) d'acides carboxyliques, on utilise l'acétate de mercure (I) et/ou de mercure (II) et/ou le benzoate de mercure (II) et/ou le trifluoracétate de mercure (II).

4. Procédé selon les revendications 1 à 3, caractérisé par le fait que comme sels minéraux dissociables de mercure (I) et/ou de mercure (II), on utilise le nitrate de mercure (II) et/ou le sulfate de mercure (I).

5. Procédé selon les revendications 1 à 4, caractérisé par le fait que comme solvants aprotiques dipolaires, on utilise le diméthylformamide, le diméthylacétamide, le diméthylsulfoxyde et/ou l'hexaméthylphosphorotriamide.

6. Procédé selon les revendications 1 à 4, caractérisé par le fait que comme solvants basiques, on utilise une ou plusieurs amines tertiaires, en particulier la triéthylamine.

7. Procédé selon les revendications 1 à 6, caractérisé par le fait que l'on conduit la réaction des dérivés 17α-éthynyl-17β-trifluoracétoxygon-4-ène-3-one de formule générale II sur l'acide formique ou l'acide acétique entre 20 et 80 °C.

8. Procédé selon les revendications 1 à 7, caractérisé par le fait que l'on conduit la réaction des dérivés 17α-éthynyl-17β-trifluoracétoxygon-4-ène-3-one de formule générale II sur l'acide formique ou l'acide acétique entre 45 et 55 °C.

9. Procédé selon les revendications 1 à 8, caractérisé par le fait que comme dérivés 17α-éthynyl-17β-trifluoracétoxygon-4-ène-3-one de formule générale II, on utilise in situ ceux qui sont contenus dans les mélanges réactionnels obtenus lors de leur préparation en partant des dérivés 17α-éthynyl-17β-hydroxygon-4-ène-3-one correspondants.

10. Dérivés 17α-éthynyl-17β-trifluoracétoxygon-4-ène-3-one de formule générale II

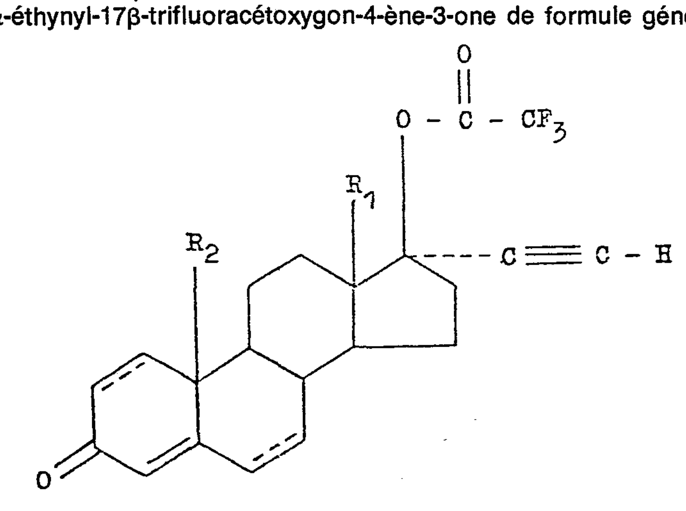

(II)

dans laquelle

$R_1$ représente un radical méthyle ou éthyle,

$R_2$ représente l'hydrogène ou un radical méthyle.

les tiretés (----) représentent des liaisons chimiques supplémentaires existant éventuellement.

11. La 17α-éthynyl-17β-trifluoracétoxyandrost-4-ène-3-one.
12. La 17α-éthynyl-17β-trifluoracétoxyandrosta-1,4-diène-3-one.
13. La 17α-éthynyl-17β-trifluoracétoxyandrosta-1,4-6-triène-3-one.
14. La 17α-éthynyl-17βtrifluoracétoxy-18-méthyl-estr-4-ène-3-one.
15. La 17α-éthynyl-17β-trifluoracétoxyestr-4-ène-3-one.
16. Médicaments caractérisés par une teneur en un ou plusieurs composés selon les revendications 10 à 15 comme substances actives, avantageusement en même temps qu'un ou plusieurs agents de conditionnement pharmaceutiques usuels.